Europäisches Patentamt

European Patent Office

Office européen des brevets

(19)

(11) Publication number: **0 446 100 A2**

(12) **EUROPEAN PATENT APPLICATION**

(21) Application number: 91400516.0

(22) Date of filing: 26.02.91

(51) Int. Cl.⁵: **G01N 33/68, // C12P21/08**

(30) Priority: 27.02.90 IT 4153790

(43) Date of publication of application:
11.09.91 Bulletin 91/37

(84) Designated Contracting States:
AT BE CH DE DK ES FR GB GR IT LI LU NL SE

(71) Applicant: FIDIA S.p.A.
Via Ponte della Fabbrica 3-A
I-35031 Abano Terme (Padova) (IT)

(72) Inventor: Della Valle, Francesco
Via Cerato 14
Padova (IT)
Inventor: Callegaro, Lanfranco
Via Bravi 35
I-35020 Ponte di Branta, Padova (IT)

(74) Representative: Hirsch, Marc-Roger
Cabinet Hirsch 34 rue de Bassano
F-75008 Paris (FR)

(54) **Method for the quantitative determination of the biologically active form of the human nerve growth factor in protein liquids.**

(57)    The present invention relates to a sandwich-type method for the quantitative determination of the biologically active form of the human nerve growth factor (NGF), using polyclonal and monoclonal antibodies specific to the biologically active human form of NGF, defined as 2.5S or βNGF.

EP 0 446 100 A2

# FIG.I

KD

94.0

6 7.0

4 3.0

30.0

20.0

1 4.4

# METHOD FOR THE QUANTITATIVE DETERMINATION OF THE BIOLOGICALLY ACTIVE FORM OF THE HUMAN NERVE GROWTH FACTOR IN PROTEIN LIQUIDS

## BACKGROUND OF THE INVENTION

Nerve growth factor (NGP) was originally discovered in mouse sarcoma (Levi-Montalcini R. et al., J. Exp. Zool. 116:321, 1951) and was subsequently purified to homogeneity from submandibular salivary glands of male mice (Varon S. et al., Biochemistry 6:2202, 1967) and from snake venom (Angeletti R. H., Proc. Natl. Acad. Sci. USA 65:668, 1970). Many other relatively rich sources of NGP have been reported, such as guinea pig prostate (Harper G. P. et al., Nature 279:160, 1979) and human placenta (Goldstein L. D. et al., Neurochem. Res. 3:175, 1978; Walker P. et al., Life Science 26:195, 1980; Calissano P. et al., Hormonal Prot. Peptides, XII:2, 1984; Italian patent application No. 47745A88; Callegaro et al., Proceedings of the Satellite to the 20th Annual Meeting of the American Society for Neurochemistry "Trophic Factors and the Nervous System", Columbus, Ohio, March 12-14, 1989, Raven Press, Fidia Research Series - FIDIA Research Foundation Symposium Series Vol. 3, Ed. L.A. Horrocks et al., pg. 75 (1990)). Small quantities of NGF have been found in other tissues, such as in the mammalian central nervous system (Varon S., Discussions in Neuroscience, Vol. II, n. 3, 1985; Hefti F., et al., Neuroscience 14:55, 1985). The physiological correlation between these potential sources of NGF and its apparent action sites are not too clear, but it is generally believed that NGF is secreted from the various peripheral tissues which require innervation by those cells which respond to NGF.

NGF from male mouse submandibular salivary glands has also been sequenced and cloned (Scott J. et al., Nature 302:538, 1983; Ulrich A. et al., Nature 303:821, 1983). The human βNGP gene also has been successfully isolated and cloned (Ulrich A. et al., Nature 303:821, 1983; European patent No. 0121388; Italian Patent Application No. 48564A89).

NGF from mouse submandibular glands is the type most widely used to study the activity of NGF in vitro and in vivo. The range of NGF's biological activity in vitro has been determined both on primary neuronal cells and on cloned cell lines. Among the primary neuronal cells which responded to NGF in vitro are fetal sensory neurons (fetal days 8-12) from spinal ganglion root, autonomic, noradrenergic, fetal neurons from sympathetic ganglia, cholinergic fetal neurons from the septum and chromaffin adrenal cells during development. While the sensory and sympathetic neurons depend on NGF for survival and development, the cholinergic neurons do not seem to need NGF for survival but only for differentiation, that is, for the expression of their characteristic phenotype features bound to the neurotransmitter. The addition of NGF to chromaffin adrenal cells (derived from the neuronal crest) during the first stage of their development causes the expression of neuronal phenotypes. According to the literature, the cell lines which respond to NGF in vitro include chromaffin adrenal cells derived from neuronal crest tumour, and known as pheochromocytoma (PC12) cells and human neuroblastoma cells. After treatment with βNGF these cells behave differently, passing from a highly proliferative phase to a postmitotic condition.

Numerous in vitro and in vivo studies on animal models have been carried out and research has now advanced so as to justify the hypothesis of a potential use of NGF in the treatment of Parkinson's disease (Science 243:11, 1989). Recently, other extremely important hypotheses have been put forward with NGF playing a mediatory role between the central nervous system and the immune system (Aloe L. et al., Proc. Natl. Acad. Sci. USA 1983, 6184:1986; Spillantini M.G. et al., Proc. Natl. Acad. Sci. USA 1986, 8555:1989). From these writings emerges the need for a quantitative, analytical methodology for the determination of human NGF in biological fluids or culture mediums. Suitable analytical methods and specific reagents, for the molecules to be analyzed, are essential.

The method according to the present invention recognizes specific epitopes and therefore does not give false negative results.

## SUMMARY OF THE INVENTION

The invention is directed to a method for the quantitative determination of the 2.5S form of human NGF which comprises:

(a) contacting a sample of biological fluid to be detected with antianalyte antibodies which recognize human NGF and which are bound to or absorbed onto a solid support to form a solid phase;

(b) separating said solid phase from said sample of biological fluid;

(c) incubating said solid phase in the presence of a detergent and monoclonal antibodies which recognize and bond to said 2.5S form of human NGF; and

(d) determining the amount of said 2.5S form of human NGF by quantitatively measuring the amount of said monoclonal antibody bound to said 2.5S form of human NGF.

A further aspect of the invention is directed to a kit for determining the 2.5S form of human NGF which comprises:

(a) antianalyte antibodies bound to a solid support; and

(b) monoclonal antibodies specific to said 2.5S form of NGF.

## BRIEF DESCRIPTION OF THE DRAWINGS

Figure 1 is a Western Blot after reaction of human or mouse NGF with polyclonal antibodies produced in rabbit.

Figure 2 is a Western Blot after reaction of human or mouse NGF with a specific monoclonal antibody for the 7.5S mouse form.

Figure 3 is an ELISA core response cure using various concentrations of hNGF in its 2.5S or βNGP form obtained from enkaryotic cells (CHO) by recombinant DNA technology.

## DETAILED DESCRIPTION OF PREPERRED EMBODIMENTS

NGF from murine glands acts as a 7S-type protein complex (molecular weight about 140,000 dalton) composed of three different subunits ($\alpha$, $\beta$, $\gamma$) coordinating a Zn+ atom. The most interesting part of the 7S molecule, in terms of biological activity, is constituted by two polypeptide chains, each with a molecular weight of 13,250 and formed by 118 amino acids. Each chain or monomer has three disulfide bridges, which form covalent bonds between two cysteine residues, conferring strong stability to the three-dimensional structure of the protein. The two NGF monomers, joined by weak bonds, form a dimer having a molecular weight of 26,500. It has been demonstrated that the molecule's biological activity is associated with the dimer known as 2.5S, or more conventionally βNGF. Genetic engineering techniques have made it possible to identify the gene which encodes the molecule of the β-subunit of NGF (βNGF) (Scott J. et al, Nature 302:538, 1983; Ullrich A. et al, Nature 303:821, 1983). The human gene encoding the molecule is to be found in the short arm of chromosome I and encodes by the synthesis of a much bigger molecule than that with a molecular weight of 26,500 which is the biologically active molecule. The gene therefore transmits instructions for the synthesis of a larger NGF or pro-NGF precursor. It has been demonstrated that the gene of the NGF β-subunit is well conserved in various species, from birds to humans (Meier R. et al, EMBO J. 5:1489, 1986). By sequencing the nucleotides of mouse, human, bovine and chick βNGF, it has been possible to compare the conserved and non-conserved sites of this molecule and their amino acid relationships. Of all analytical methods, the sandwich system is surely the most advanced in terms of sensitivity and specificity.

Determination of an analyte in biological fluid by a sandwich-type reaction system, is done by incubating the sample, with or without the analyte, with a support to which antianalyte antibodies are bound, generally defined as the solid phase. After eliminating the unreacted analyte which has not reacted with bound antianylate antibodies, the solid phase is washed with suitable buffers and incubated with antibodies specific to the analyte in question. These antibodies can be directly labelled with a visualizing agent or they can be identified by other agents, such as antispecies antibodies, where antibodies against analytes of the second incubation have been produced, labelled with suitable tracers. Numerous identification methodologies are now available, allowing the visualization of an interaction between analyte and antibody in the sandwich system, and in the same way there are many supports to which antibodies can be bound. Apart from improvements in supports and tracer systems which are part of the overall technological advancement, the use of a quantitative, analytical dosage by a sandwich system is fundamentally based on its specificity, that is, on its infallible ability to detect the presence of the analyte in a fluid. Clearly, if the analyte is recognized only when it has a well-defined structural form, such as that which is associated with its biological activity, this increases the specificity characteristics of the analytical dosage and consequently the biological significance of the quantitative determination of the analyte in question. Specificity in a sandwich-type analytical dosage is mainly guaranteed by the antianalyte antibodies.

In the present invention, the analyte is NGF and the goal of the invention is an assay for the quantitative determination of human NGF in its mature, biologically active form generally known as 2.5S or βNGF. The abbreviated name, NGF, also defines the biologically active form of the molecule.

The anti-NGF antibodies used in the solid phase, that is, absorbed onto the solid support, were produced by immunizing rabbits with mouse NGF and purified by affinity chromatography on the same protein, immobilized on a solid support (K. Stoeckel et al., J. Neurochem., 26:1207, 1976), belonging to a different preparation from the one used for immunization. These polyclonal anti-mouse NGF antibodies, were assessed for crossreactivity with human NGF extracted from human placenta (European Patent Publication No. 333,574) and with human NGF in its biologically active form, 2.5S or βNGF, cloned and expressed in prokaryotic cells such as E. coli or in eukaryotic cells (Italian Patent Application No. 48564A89 and European Patent Publication No. 0121388) and also in its denatured form as a linear polypeptide.

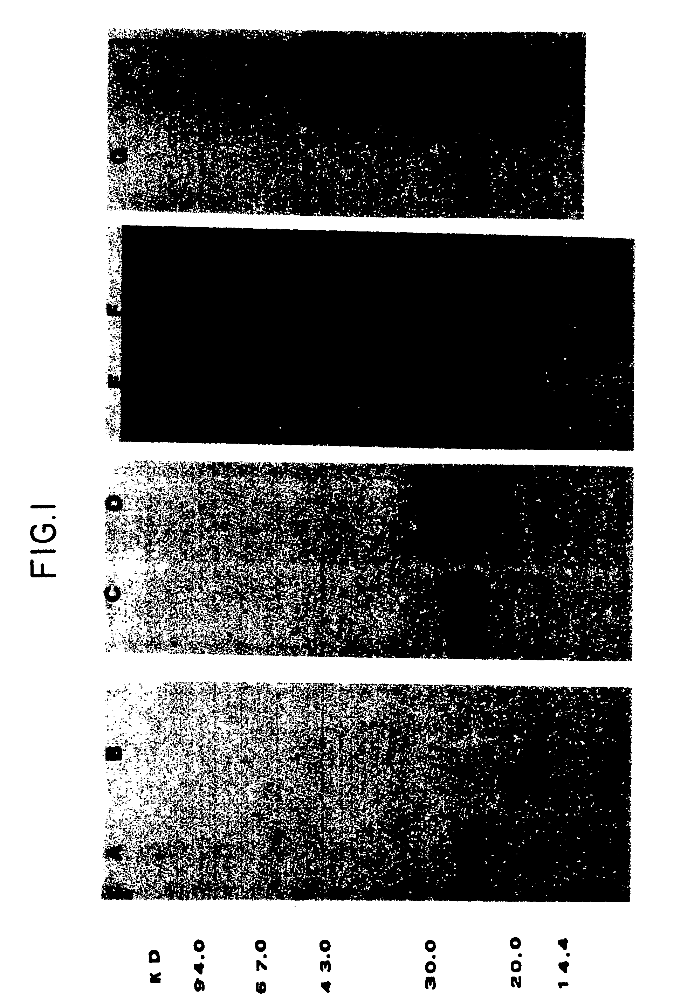

The reactivity of these anti-NGF polyclonal antibodies was assessed by the well-known Western Blot technique. The results appear in Figure 1.

Figure 1 is an example of Western Blot after reaction of human or mouse NGF with polyclonal antibodies, produced in rabbit by immunization with the 2.5S form of mouse NGF and purified by affinity chromatography in the same protein immobilized on a solid support. The presence of polyclonal antibodies was detected using a commercial amplification system.

The different lanes were:

Lane A: mNGF in its dimeric form, 2.5S or βNGF.

Lane B: mNGF in its monomeric form after incubation with βmercaptoethanol 1mM for 1 minute at 100°C.

Lane C: mNGF in the form described in lane A.

Lane D: hNGF in its 2.5S form, obtained by the recombinant DNA technique in eukaryotic cells (Chinese Hamster Ovary).

Lane E, Lane F: hNGF in its 2.5S form, purified from human placenta tissue.

Lane G: purified hNGF, in its 2.5S form, obtained from prokaryotic cells (E. Coli) by the recombinant DNA technique.

Lane H: mNGF in the form described in lane A and lane C.

The standard molecular weight indicators used were: phosphorylase B (molecular weight 94,000), albumin (molecular weight 67,000), ovalbumin (molecular weight 43,000), carbonic anhydrase (molecular weight 30,000), tripsin inhibitor (molecular weight 20,000), alpha-lactoalbumin (molecular weight 14,400).

In the same way, it is possible to obtain anti-mouse NGF monoclonal antibodies by immunizing rats of particular species (Cattaneo A. et al., J. Neurochem. 50:1003, 1988). If properly selected, it is possible also to identify monoclonal antibodies which recognize NGF only in its biologically active form, 2.5S or βNGF.

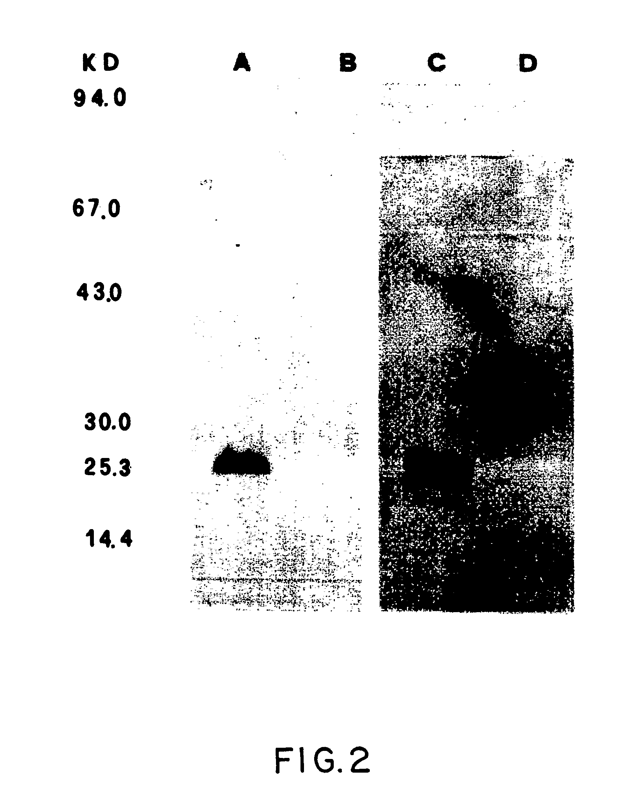

When developing the sandwich assay of the present invention, a monoclonal antibody presenting these characteristics and which unequivocally recognized human NGF, partially purified from placenta (European Patent Publication No. 333,574) or obtained by recombinant DNA technology (See e.g., Italian Patent Application No. 48564A89 and European Patent Publication No. 0121388), in its 2.5S form was used. Figure 2 shows the results of characterization of the monoclonal antibody by the Western Blot technique. Moreover, the monoclonal anti-NGF antibody, the characterization data of which are shown in Figure 2, blocked the biological activity of mouse NGF (Cattaneo A. et al., J. Neurochem. 50:1003, 1988). Characterization of antibodies shows that they recognize the biologically active form of human NGF and consequently have the necessary specificity for the development of a specific sandwich-type dosage for human NGF.

Figure 2 is an example of Western Blot after reaction of human or mouse NGF with a specific monoclonal antibody for the 2.5S mouse form. The presence of the monoclonal antibody can be revealed by commercial reagents.

The different lanes were:

Lane A: mNGF in its dimeric form 2.5S or βNGF. Lane B: mNGF in its monomeric form, obtained as described in Figure 1 (lane B).

Lane C: hNGF in its 2.5S form, purified from human placenta tissue.

Lane D: hNGF purified from human placenta and reduced to its monomeric form as described in Figure 1 (lane B).

The standard molecular weight indicators are reported in Figure 1.

The present invention also concerns a kit containing the components used in the method of the present invention. This kit must include at least those anti-NGF antibodies bound to the support and those used to detect the presence of NGF, the latter being labelled, or not labelled, as the case may be.

Possible supports to be used are, for example, tubes, microtitration plates, strips, beads or discs in glass, plastic or other suitable substances and are well known in the art.

Labelling agents which are useful may be enzymes, fluorescent compounds, staining compounds, metals or their derivatives, or antispecies antibodies labelled with the said agents and are well known in the art. A kit according to the present invention may also contain optional agents for the determination of the labelling agents, for example substrates for the enzyme, washing or dilution buffers with or without detergent.

The invention is illustrated hereinafter by Examples 1 and 2 and concerns an assay for the quantitative determination of the biologically active form of human NGF obtained by the recombinant DNA technique (e.g., Italian Patent Application No. 48564A89 and European Patent Publication No. 0121388).

**Example 1** - Determination of human NGF in culture medium on polycarbonate microtitration plates.

**A. Methods**

**A.1. Coating of the microtitration plates.**

Anti-NGF polyclonal antibodies, diluted in a bicarbonate buffer with pH 9.0 by incubation for 24 hrs at room temperature, were absorbed inside the wells of the polycarbonate microtitration plate. The wells were then washed with 0.15 moles/litre of phosphate buffer, pH 7.2.

**A.2. Incubation of the microtitration plates with the sample to be analyzed.**

50 µl of sample to be analyzed, at various concentrations, were scattered in the well of the microtitration plate and incubated for 24 hrs at room temperature. The unreacted excess sample was then discarded and the wells were washed with 0.15 moles/litre of phosphate buffer, pH 7.2, containing 2% bovine albumin and a non-ionic detergent such as 0.05% Tween 20.

**A.3. Incubation with anti-NGF monoclonal antibodies.**

The wells of the microtitration plate prepared as described in A.2. were filled with 50 µl of a solution of said anti-NGF monoclonal antibodies, diluted in a phosphate buffer 0.15M, pH 7.2, containing 2% bovine albumin and incubated for 24 hrs at room temperature. The excess non-reacted reagent was discarded, the wells were washed with 0.15M phosphate buffer, pH 7.2, containing 0.05 % of Tween 20 and 2% of bovine albumin.

**A.4. Incubation with labelled antispecies antibody.**

The wells obtained by the procedure described in A.3. were filled with 50 µl of a commercial tracer containing anti-rat IgG goat IgG labelled with an enzyme, such as horseradish peroxidase. Incubation lasted from 4 to 6 hrs at room temperature or at 37°C. The excess reagent was then discarded by washing with phosphate buffer 0.15M, pH 7.2.

**A.5. Incubation with substrate.**

The wells obtained as described in A.4. were filled with 50 µl of a mixture of 0.4 mg/ml of orthophenylenediamine and 0.2 mg/ml of hydrogen peroxide in a buffer with pH 5.0. After incubation for 15 minutes, 50 µl of sulfuric acid, 2.5 moles/litre were added to the wells of the microtitration plate.

**A.6. Absorbency measurement.**

The absorbances of the solutions present in the wells of the microtitration plates were measured with a photometer at a wavelength of 492 mm (OD 492 mm).

**B. Results**

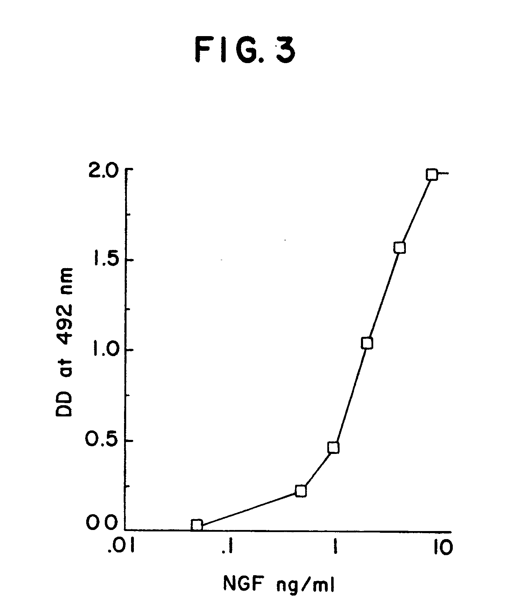

Absorbance of the wells containing human NGF at various concentrations, in the culture medium, examined by the aforesaid method, are reported in Figure 3.

**EXAMPLE 2** - Test kit

To determine human NGF as described in Example 1 we used a multidosage kit containing:
- polycarbonate microtitration plates with wells absorbed with polyclonal antibodies which recognize NGF in its form known as 2.5S or βNGF and in its non-dimeric forms;
- ampoules containing monoclonal antibody which specifically recognizes the human biologically active form 2.5S or βNGF;
- ampoules containing enzymatic tracers specific for the species where the anti-NGF monoclonal antibody has been produced;
- bottles of phosphate buffer, pH 7.2, containing bovine albumin;
- bottles containing solutions of Tween 20 detergent;

– bottles of substrate containing orthophenylenediamine and hydrogen peroxide in buffer at pH 5.0;
– ampoules containing solutions, at various concentrations, of human NGF, also including a negative control solution;
– instructions for the correct use of the test.

## Claims

1. A method for the quantitative determination of the 2.5S form of human NGF which comprises:
   (a) contacting a sample of biological fluid to be detected with antianalyte antibodies which recognize human NGF and which are bound to or absorbed onto a solid support to form a solid phase;
   (b) separating said solid phase from said sample of biological fluid;
   (c) incubating said solid phase in the presence of monoclonal antinodies which recognize and bond to said 2.5S form of human NGF; and
   (d) determining the amount of said 2.5S form of human NGF by quantitatively measuring the amount of said monoclonal antibody bound to said 2.5S form of human NGF.

2. The method according to Claim 1, wherein said antianalyate antibodies are polyclonal antibodies which recognize human NGF.

3. The method according to Claim 1, wherein said monoclonal antibodies are labeled with an enzyme.

4. The method according to Claim 1, wherein said antianalyte antibodies are bound to a solid support.

5. The method according to Claim 1, wherein said solid support is polycarbonate.

6. The method according to Claim 1, wherein said monoclonal antibodies are incubated with said solid support in the additional presence of a non-ionic detergent.

7. The method according to Claim 6, wherein said non-ionic detergent is Tween 20®

8. A kit for determining the 2.5S form of human NGF which comprises
   (a) antianalyte antibodies bound to a solid support; and
   (b) monoclonal antibodies specific to said 2.5S form of NGF.

9. The kit according to Claim 8, further comprising labelled monoclonal antibodies specific to the 2.5S form of NGF.

10. The method according to Claim 9, wherein said label is an enzyme.

11. The kit according to Claim 10, further comprising a substrate for said enzyme.

12. The kit according to Claim 8, wherein said solid support is a polycarbamate microtitration plate.

13. The kit according to Claim 8, further comprising a standard curve of human NGF.

14. The kit according to Claim 8, further comprising a protein solution containing a detergent.

15. The method according to Claim 2, wherein said human NGF is human placental NGF.

16. The method according to Claim 1, wherein said monoclonal antibodies are specific to said 2.5S form of human placental NGF.

# FIG.I

EP 0 446 100 A2

FIG.2

# FIG. 3